(19) Europäisches Patentamt
European Patent Office
Office européen des brevets

(11) Veröffentlichungsnummer : **0 603 131 A1**

(12) **EUROPÄISCHE PATENTANMELDUNG**

(21) Anmeldenummer : **93810861.0**

(22) Anmeldetag : **09.12.93**

(51) Int. Cl.[5] : **C07C 275/40,** C08G 59/68, C08G 59/40

(30) Priorität : **18.12.92 CH 3893/92**

(43) Veröffentlichungstag der Anmeldung : **22.06.94 Patentblatt 94/25**

(84) Benannte Vertragsstaaten : **CH DE ES FR GB IT LI NL SE**

(71) Anmelder : **CIBA-GEIGY AG**
**Klybeckstrasse 141**
**CH-4002 Basel (CH)**

(72) Erfinder : **Mühlebach, Andreas, Dr.**
**Les Grands Esserts 7**
**CH-1782 Belfaux (CH)**
Erfinder : **Flury, Peter, Dr.**
**Muldenweg**
**CH-4204 Himmelried (CH)**

(54) **Aromatische Harnstoffverbindung als Härtungsbeschleuniger für eine Mischung aus Epoxiharz und Dicyandiamid.**

(57) Beschrieben wird die Verwendung einer Verbindung der Formel 1

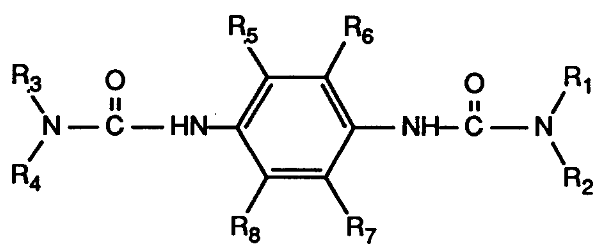

(1),

worin $R_1$ und $R_3$ unabhängig voneinander jeweils eine Alkyl-, Cycloalkyl- oder Arylgruppe, $R_2$ und $R_4$ unabhängig voneinander jeweils eine Alkyl- oder Arylgruppe oder $R_1$ mit $R_2$ und/oder $R_3$ mit $R_4$ jeweils eine 1,4-Tetramethylen- oder eine 1,5-Pentamethylengruppe und schliesslich $R_5$, $R_6$, $R_7$ und $R_6$ unabhängig voneinander jeweils ein Wasserstoff- oder Halogenatom oder eine $C_1$-$C_4$-Alkyl- oder $C_1$-$C_4$-Alkoxygruppe bedeuten, als Härtungsbeschleuniger für eine heisshärtende Zusammensetzung enthaltend mindestens ein Epoxidharz und Dicyandiamid, entsprechende Zusammensetzungen sowie zwei vorteilhafte Verfahren zur Herstellung von Verbindungen der Formel 1. Die Beschleuniger der Formel 1 haben den Vorteil, dass sie zwar die Reaktivität der Zusammensetzungen bei erhöhter Temperatur in vergleichbarem Masse wie gute Beschleuniger des Standes der Technik erhöhen, die Lagerstabilität der Zusammensetzungen bei Raumtemperatur aber nicht nachteilig beeinflussen.

EP 0 603 131 A1

Die vorliegende Erfindung betrifft die Verwendung von Verbindungen der weiter unten angegebenen Formel 1 als Härtungsbeschleuniger für heisshärtende Zusammensetzungen enthaltend mindestens ein Epoxidharz und Dicyandiamid, entsprechende heisshärtende Zusammensetzungen sowie zwei zweckmässige Verfahren zur Herstellung von Verbindungen der Formel 1.

Dicyandiamid ist ein bekannter und häufig eingesetzter Härter für Epoxidharze (Henry Lee, Kris Neville "Handbook of Epoxy Resins", McGraw-Hill Book Company, New York, 1967, S. 10-16). Es ist nämlich bei Raumtemperatur oder mässig erhöhten Temperaturen bis etwa 40 °C nur sehr wenig reaktiv und ermöglicht so eine lange Handhabung und/oder Lagerung von Zusammensetzungen aus Epoxidharzen und Dicyandiamid bei diesen Temperaturen, ohne dass eine teilweise Härtung die Verarbeitbarkeit der Zusammensetzungen, beispielsweise aufgrund erhöhter Viskosität, erschwert oder gar unmöglich macht. Allerdings erfordert Dicyandiamid auch relativ hohe Temperaturen für die Härtung, zumindest oberhalb von 145 °C, insbesondere wenn, wie z.B. bei Klebstoffanwendungen oftmals der Fall, eine schnelle Härtung erforderlich ist.

Um die Reaktivität derartiger Zusammensetzungen zu steigern, werden daher üblicherweise Härtungsbeschleuniger zugesetzt. Als besonders gute Härtungsbeschleuniger sind Imidazolderivate und insbesondere N'-(3-Chloro-4-methylphenyl)-N,N-dimethylharnstoff (Chlortoluron) bekannt. Chlortoluron vermag die Reaktivität von Epoxidharz/Dicyandiamidzusammensetzungen ganz beträchtlich anzuheben. So liegt die Gelierzeit von chlortoluronbeschleunigten Epoxidharz/Dicyandiamidgemischen bei 160 °C nur bei einigen wenigen Minuten, während ohne den Härtungsbeschleuniger bei gleicher Temperatur bis zur Gelierung einer entsprechenden Zusammensetzung etwa eine halbe bis eine Stunde vergehen. Chlortoluron und andere vergleichbar gute Beschleuniger haben aber den Nachteil, dass sie die Reaktivität der Reaktionsgemische bei Raumtemperatur und mässig erhöhter Temperatur ebenfalls so drastisch erhöhen, dass die Lagerfähigkeit dieser Zusammensetzungen nicht mehr befriedigen kann.

Es bestand daher die Aufgabe, einen Härtungsbeschleuniger für heisshärtende Zusammensetzungen auf Basis von Epoxidharzen und Dicyandiamid anzugeben, der zwar bei erhöhter Temperatur vergleichbare Härtungsgeschwindigkeiten wie Chlortoluron ermöglicht, der jedoch bei Raumtemperatur oder mässig erhöhter Temperatur nur geringe Reaktivität zeigt, so dass sich bei diesen Temperaturen die Verarbeitbarkeit der Zusammensetzungen auch während einer längeren Standzeit oder Lagerung nicht oder nur unwesentlich verändert.

Überraschend hat sich nunmehr gezeigt, dass diese Aufgabe durch die Verwendung einer Verbindung der Formel 1 als Härtungsbeschleuniger für eine heisshärtende Zusammensetzung enthaltend mindestens ein Epoxidharz und Dicyandiamid gelöst werden kann

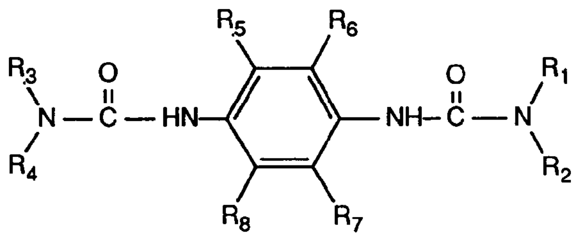
(1),

wobei in Formel 1 die Reste $R_1$ und $R_3$ unabhängig voneinander jeweils eine Alkyl-, Cycloalkyl- oder Arylgruppe, die Reste $R_2$ und $R_4$ unabhängig voneinander jeweils eine Alkyl- oder Arylgruppe oder der Rest $R_1$ zusammen mit dem Rest $R_2$ und/oder der Rest $R_3$ zusammen mit dem Rest $R_4$ jeweils eine 1,4-Tetramethylen- oder eine 1,5-Pentamethylengruppe und schliesslich die Reste $R_5$, $R_6$, $R_7$ und $R_6$ unabhängig voneinander jeweils ein Wasserstoff- oder Halogenatom oder eine $C_1$-$C_4$-Alkyl- oder $C_1$-$C_4$-Alkoxygruppe bedeuten.

In der Bedeutung von Alkyl stellen die Reste $R_1$ bis $R_4$ in Formel 1 bevorzugt Alkylgruppen mit 1 bis 6 Kohlenstoffatomen dar, die geradkettig oder verzweigt sein können, z. B. Methyl-, Ethyl- Propyl-, iso-Propyl, n-Butyl-, sec-Butyl-, tert-Butyl-, iso-Butyl- oder geradkettige oder entsprechend verzweigte Pentyl- oder Hexylgruppen dar. In der Bedeutung Cycloalkyl stellen sie insbesondere Cyclopentyl- oder Cyclohexylgruppen und in der Bedeutung Aryl insbesondere Phenylgruppen dar, oder Reste, die sich von den genannten Cycloalkyl und von Phenylgruppen durch die Substitution eines oder mehrerer, z. B. zweier oder dreier Wasserstoffatome durch Halogenatome, insbesondere Chlor- oder Bromatome, oder durch geradkettige oder verzweigte Alkylgruppen mit 1, 2, 3 oder vier Kohlenstoffatomen oder durch diesen Alkylgruppen entsprechende Alkoxygruppen ableiten.

Die Verbindungen der Formel 1 sind zumindest teilweise bekannt und können z. B. in an sich ebenfalls bekannter Weise durch gleichzeitige Umsetzung eines Diamins der Formel

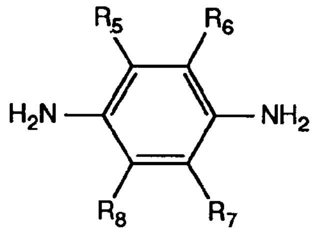

mit einem Amin der Formel $HNR_1R_2$ sowie einem Amin der Formel $HNR_3R_4$ in Gegenwart von Kohlenmonoxid und Schwefel im Autoklaven erhalten werden. Den Resten $R_1$ bis $R_8$ kommt in den vorgenannten Formeln die gleiche Bedeutung wie in Formel 1 zu. Die Mengen der Reaktanten sind nicht kritisch, die Reaktanten sollten aber im allgemeinen zumindest in den stöchiometrischen Mengen vorliegen. Dieses Verfahren ist an Hand einer Vielzahl von Beispielen im einzelnen in der US-A-2,993,044 beschrieben.

Weiterhin haben sich zwei neue Einstufenverfahren für die Herstellung von Verbindungen der Formel 1 als gangbar erwiesen. Diese bilden ebenfalls einen Gegenstand der vorliegenden Erfindung. Beide Verfahren machen den Einsatz eines Druckautoklaven unnötig und führen nicht zur Bildung von Schwefelwasserstoff wie das oben beschriebene vorbekannte Herstellungsverfähren, so dass eine Schwefelwasserstoffabsorption unnötig ist.

Beim ersten dieser Einstufenverfahren setzt man eine Diisocyanatverbindung der Formel

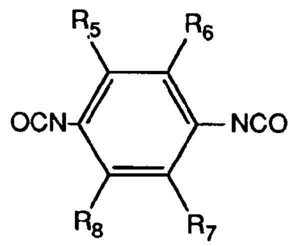 ,

worin $R_5$, $R_6$, $R_7$ und $R_8$ die gleiche Bedeutung haben, wie oben schon angegeben, mit jeweils zumindest etwa stöchiometrischen Mengen der Amine $HNR_1R_2$ und $HNR_3R_4$, worin die Reste $R_1$ bis $R_4$ ebenfalls die oben schon genannte Bedeutung haben, in einem geeigneten gegenüber Isocyanaten inerten Lösungsmittel, z. B. in Essigester, Dioxan oder einem geeigneten Kohlenwasserstoff, unter wasserfreien Bedingungen und unter Erwärmung um und isoliert das Produkt der Formel 1. Zur Isolation kann man z. B. das Lösungsmittel abziehen. Manche der Verbindungen der Formel 1 sind jedoch so schwer in den genannten Lösungsmitteln löslich, dass sie allein durch Filtration in hervorragender Ausbeute isoliert werden können. Dies trifft insbesondere für die Verbindungen der Formel 1 zu, in denen die Reste $R_1$ bis $R_4$ alle einen, vorzugsweise den gleichen Alkylrest mit 1 bis 3 Kohlenstoffatomen darstellen. Das geschilderte Verfahren führt im allgemeinen zu annähernd hundertprozentigen Ausbeuten an gewünschtem Produkt und liefert dieses in grosser Reinheit.

Das zweite Verfahren geht wie das oben beschriebene Autoklavenverfahren von einem Diamin der Formel

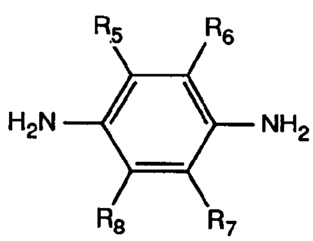

aus, das mit zumindest etwa stöchiometrischen Mengen der Carbamoylchloride der Formeln

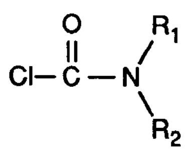 und 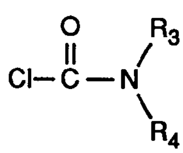

in einem geeigneten Lösungsmittel umgesetzt wird. Danach wird das Produkt von Formel 1 isoliert. Die Reaktion verläuft im allgemeinen schwach exotherm.

Einen weiteren Gegenstand der Erfindung bilden heisshärtende Zusammensetzungen enthaltend mindestens ein Epoxidharz, Dicyandiamid und einen Härtungsbeschleuniger, die dadurch gekennzeichnet sind, dass als Härtungsbeschleuniger eine Verbindung der Formel 1 in der vorgenannten Bedeutung eingesetzt wird.

Die erfindungsgemässen Zusammensetzungen zeigen bei erhöhter Temperatur eine ähnliche Reaktivität wie Zusammensetzungen auf Basis von Chlortoluron als Härtungsbeschleuniger, weisen jedoch gleichzeitig eine unerwartet stark gesteigerte Stabilität bei Raumtemperatur im Vergleich zu den genannten Zusammensetzungen auf, die vergleichbar ist mit der Stabilität entsprechender Zusammensetzungen ohne Härtungsbeschleuniger.

Besonders bevorzugt sind die Zusammensetzungen auf Basis derjenigen Härtungsbeschleuniger von Formel 1, bei denen die Reste $R_1$, $R_2$, $R_3$ und $R_4$ in Formel 1 unabhängig voneinander jeweils eine Alkylgruppe mit 1 bis 3 Kohlenstoffatomen oder Phenyl bedeuten, sowie Zusammensetzungen auf Basis der Verbindungen von Formel 1, bei denen die Reste $R_5$, $R_6$, $R_7$ und $R_8$ alle Wasserstoffatome darstellen.

Am meisten bevorzugt sind schliesslich erfindungsgemässe Zusammensetzungen mit der Verbindung der Formel 1, bei der die Reste $R_1$, $R_2$, $R_3$ und $R_4$ alle Methyl und die Reste $R_5$, $R_6$, $R_7$ und $R_8$ alle Wasserstoffatome bedeuten.

Alle gebräuchlichen Di- und Polyepoxide sowie Epoxidharzpräpolymere sind als Epoxidharze für die Erfindung geeignet. Die Di- und Polyepoxide können aliphatische, cycloaliphatische oder aromatische Verbindungen sein. Beispiele für solche Verbindungen sind die Glycidylether und β-Methylglycidylether aliphatischer oder cycloaliphatischer Diole oder Polyole, zum Beispiel solche des Ethylenglykols, Propan-1,2-diols, Propan-1,3-diols, Butan-1,4-diols, Diethylenglykols, Polyethylenglykols, Polypropylenglykols, Glycerins, Trimethylolpropans oder 1,4-Dimethylolcyclohexans oder des 2,2-Bis-(4-hydroxycyclohexyl)-propans, die Glycidylether von Di- und Polyphenolen, beispielsweise Resorcin, 4,4'-Dihydroxydiphenylmethan, 4,4'-Dihydroxydiphenyl-2,2-propan, Novolake und 1,1,2,2-Tetrakis-(4-hydroxyphenyl)-ethan. Weitere Beispiele sind N-Glycidylverbindungen, z.B. die Diglycidylverbindungen des Ethylenharnstoffs, 1,3-Propylenharnstoffs oder 5-Dimethylhydantoins oder des 4,4'-Methylen-5,5'-tetramethyldihydantoins, oder solche wie Triglycidylisocyanurat.

Weitere Glycidylverbindungen mit technischer Bedeutung sind die Glycidylester von Carbonsäuren, insbesondere Di- und Polycarbonsäuren. Beispiele dafür sind die Glycidylester der Bernsteinsäure, Adipinsäure, Azelainsäure, Sebacinsäure, Phthalsäure, Terephthalsäure, Tetra- und Hexahydrophthalsäure, Isophthalsäure oder Trimellitsäure, oder von dimerisierten Fettsäuren.

Beispiele für von Glycidylverbindungen verschiedene Polyepoxide sind die Diepoxide des Vinylcyclohexens und Dicyclopentadiens, 3-(3',4'-Epoxicyclohexyl)-8,9-epoxy-2,4-dioxaspiro[5.5]undecan, der 3,4'-Epoxicyclohexylmethylester der 3,4-Epoxicyclohexancarbonsäure, Butadiendiepoxid oder Isoprendiepoxid, epoxidierte Linolsäurederivate oder epoxidiertes Polybutadien.

Bevorzugte Epoxidharze sind gegebenenfalls vorverlängerte Diglycidylether zweiwertiger Phenole oder zweiwertiger aliphatischer Alkohole mit 2 bis 4 Kohlenstoffatomen, insbesondere die gegebenenfalls vorverlängerten Diglycidylether des 2,2-Bis-(4-hydroxyphenyl)-propans und Bis-(4-hydroxyphenyl)-methans oder ein Gemisch aus diesen Epoxidharzen.

Obwohl gerade Zusammensetzungen auf Basis einer Harzkomponente nur aus Epoxidharzen eine wichtige Ausführungsform der Erfindung darstellen, können neben den Epoxidharzen auch noch Polyisocyanate in den härtbaren Zusammensetzungen enthalten sein.

Als Polyisocyanate sind hierbei alle zur Vernetzung fähigen Polyisocyanate geeignet, z. B. Hexamethylendiisocyanat (HDI), Trimethylhexamethylendiisocyanat (TMDI), Cyclohexandiisocyanat (CHDI), Isophorondiisocyanat (3,5,5-Trimethyl-1-isocyanato-3-isocyanatomethylcyclohexan; IPDI), Methylendicyclohexylisocyanat (HMDI), p-Phenylendiisocyanat (PPDI), Diisocyanatotoluol (TDI), z. B. 2,4-Diisocyanatotoluol, 2,6-Diisocyanatotoluol und technische Gemische von beiden Isomeren, Naphthylendiisocyanat (NDI), insbesondere 1,5-Naphthylendiisocyanat, Dianisidindiisocyanat (DADI), Methylendiphenyldiisocyanat (MDI), insbesondere das 4,4'-Isomere, aber auch technische Gemische verschiedener Isomere, beispielsweise der 4,4'- und 2,4'-Isomeren, oder Polymethylenpolyphenylisocyanate (PAPI). Ebenfalls gut geeignet sind auch Polyisocyanate, die durch Umsetzung von Polyisocyanaten mit sich selbst über Isocyanatgruppen erhältlich sind, wie Uretdione oder Carbodiimidverbindungen, die durch Reaktion zweier Isocyanatgruppen entstehen, oder wie Isocyanurat- oder Biuretverbindungen, die durch Reaktion dreier Isocyanatgruppen entstehen. Für die Erfindung geeignet sind auch Polyisocyanatpräpolymere, die durchschnittlich mehr als eine Isocyanatgruppe pro Molekül aufweisen und durch Vorreaktion eines molaren Überschusses beispielsweise eines der oben genannten Polyisocyanate mit einem organischen Material erhalten werden, das mindestens zwei aktive Wasserstoffatome pro Molekül aufweist, z. B. in Form von Hydroxylgruppen wie bei Polyalkylenglykolen. Isocyanate wie die genannten sind allgemein verfügbar und in grosser Vielfalt kommerziell erhältlich.

Bevorzugt werden als Polyisocyanate Methylendiphenyldiisocyanat (MDI), Isophorondiisocyanat (IPDI), Hexamethylendiisocyanat, Diisocyanatotoluol (TDI), z. B. 2,4-Diisocyanatotoluol, 2,6 Diisocyanatotoluol und technische Gemische von beiden Isomeren, und Präpolymere der genannten Isocyanate mit Polyalkoholen sowie Umsetzungsprodukte, die durch Umsetzung der Polyisocyanate mit sich selbst über Isocyanatgruppen erhältlich sind.

Die freien Isocyanatgruppen der Polyisocyanate können auch in üblicher Weise blockiert sein, z. B. mit Phenolen, Oximen, Caprolactamen, Imidazolen, Pyrazolen oder Indazolen. Pyrazolblockierte Polyisocyanate sind bevorzugt, weil sie bereits bei relativ niedrigen Temperaturen, ab etwa 80°C, wieder Isocyanat freisetzen und zu härten beginnen.

Pyrazolblockierte Polyisocyanate und deren Herstellung sind in der US-A-4,976,837 und der EP-A-0 500 495 beschrieben. Sie kann durch quantitative Umsetzung geeigneter Pyrazole mit den Polyisocyanaten unter Inertgas erfolgen. Vorzugsweise arbeitet man dabei bei erhöhter Temperatur und in einem geeigneten inerten Lösungsmittel (z. B. Toluol), gegebenenfalls in Gegenwart eines Katalysators (z. B. Dibutylzinndilaurat). Infolge der exothermen Reaktion beider Verbindungen kann eine Kühlung erforderlich sein. Die zur Blockierung verwendeten Pyrazole haben bevorzugt die Formel

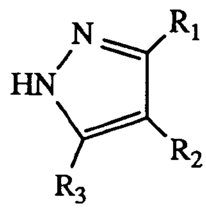 ,

wobei $R_1$, $R_2$ und $R_3$ unabhängig voneinander Wasserstoff, Hydroxyl, Alkyl, insbesondere mit einem bis zu fünf Kohlenstoffatomen, Alkyloxy, insbesondere mit einem bis zu fünf Kohlenstoffatomen, Alkylthio, insbesondere mit einem bis zu fünf Kohlenstoffatomen, wobei wiederum Methyl und Methoxy besonders bevorzugt sind; Aryl mit bis zu zehn Ringkohlenstoffatomen, insbesondere Phenyl, wobei die Arylreste gegebenfalls ihrerseits Substituenten haben können, z. B. $C_1$-$C_4$-Alkyl- oder $C_1$-$C_4$-Alkyloxysubstituenten; oder Arylalkyl, insbesondere Aryl-($C_1$-$C_4$)Alkyl, ganz besonders Arylmethyl, insbesondere Benzyl bedeuten. Pyrazole dieser Art sind im Handel oder können gemäss üblicher Methoden, z. B. durch Umsetzung passend gewählter i,i+2-Diketone, z. B. eines 1,3-Diketons, mit Hydrazin mit oder ohne Lösungsmittel (z. B. Toluol) erhalten werden.

Werden Gemische aus Epoxidharzen und Polyisocyanaten eingesetzt, so macht das Epoxidharz bevorzugt mindestens 50, noch besser mindestens 70 Gewichtsprozent der gesamten Harzkomponente aus.

Die genannten Zusammensetzungen enthalten die Verbindung der Formel 1 zweckmässigerweise in einer Menge von 0,1 bis 10 Gewichtsprozent, bezogen auf die Gesamtmenge aus Epoxidharz sowie Dicyandiamid und gegebenenfalls vorhandenem Polyisocyanat. Dicyandiamid liegt im allgemeinen in Mengen von 2 - 20 Gewichtsprozent und Epoxidharz oder Epoxidharz/Polyisocyanatgemisch in Mengen von 80 - 98 Gewichtsprozent vor, jeweils bezogen auf die Gesamtmenge aus Dicyandiamid, Epoxidharz und gegebenenfalls vorhandenem blockierten oder nicht blockierten Polyisocyanat. Als ein Beispiel mag die folgende bevorzugte Zusammensetzung dienen, die Dicyandiamid in einer Menge von etwa 10 Gewichtsprozent und 1,4-Bis-(N,N'-dimethylureido)-benzol in einer Menge von etwa 5 Gewichtsprozent enthält, bezogen auf die Gesamtmenge aus Epoxidharzen, Dicyandiamid und 1 ,4-Bis-(N,N'-dimethylureido)-benzol.

Neben den genannten Bestandteilen können die erfindungsgemässen Zusammensetzungen noch weitere übliche Bestandteile in den üblichen Mengen enthalten, wie z. B. Viskositätsregler, Streckmittel, Füllstoffe, Verstärkungsmittel, Metallpartikel, Pigmente, Farbstoffe, organische Lösungsmittel, Weichmacher, Haftvermittler, Fungizide, Antioxidantien, Verlaufsmittel, Verdünner, z. B. Reaktivverdünner und dergleichen mehr.

Die erfindungsgemässen härtbaren Gemische lassen sich ganz allgemein zur Herstellung von gehärteten Produkten einsetzen, und können in einer dem jeweils speziellen Anwendungsgebiet angepassten Formulierung beispielsweise als Beschichtungsmassen, Lacke, Pressmassen, Tauchharze, Giessharze, Imprägnierharze, Laminierharze oder Matrixharze eingesetzt werden.

Sie eignen sich z. B. zur Herstellung von Prepregs, Verbund- oder Compositematerialien, zur Herstellung von Formkörpern jeglicher Art oder zum Umhüllen von elektrischen oder elektronischen Bauteilen. Besonders gut geeignet sind die erfindungsgemässen Zusammensetzungen als 1- oder 2-Komponenten-Klebstoffe, z. B. zum Aufkleben von IC's. Ein Anwendungsgebiet speziell für die Zusammensetzungen auf Basis von Mischungen aus Epoxidharzen und Polyisocyanaten ist die Herstellung gehärteter Produkte mit einreagierten

Modifizierungsmitteln, wie Weichmachern oder Flexibilisatoren, wobei z. B. die Polyisocyanatkomponente das Modifizierungsmittel für die Epoxidharzkomponente darstellt (Interpenetrating Polymer Networks).

Besonders bevorzugt sind Anwendungen, bei denen es auf die Erreichung einer hohen Haftfähigkeit von gehärtetem erfindungsgemässen Material ankommt, d. h. die Verwendung der erfindungsgemässen Zusammensetzungen als Beschichtungsmittel, zur Herstellung von Prepregs und insbesondere als heisshärtender, bevorzugt einkomponentiger Klebstoff.

Die erfindungsgemässen Gemische können bei relativ niedrigen Temperaturen rasch ausgehärtet werden. Im allgemeinen wendet man zur Aushärtung Temperaturen im Bereich von 20 bis 200°C, vorzugsweise von 60 bis 180°C, insbesondere 80 bis 120°C, an. Die Härtung kann dabei durch Zuführ von Wärme in jeglicher Form erfolgen. Sie kann beispielsweise auch mit Hilfe von Mikrowellen oder durch Induktionserwärmung durchgeführt werden, wobei für letzteres die Zusammensetzungen selbstverständlich elektrisch leitende Partikel, z. B. Metallpartikel, enthalten müssen. Die Aushärtung erfolgt in der Regel unter gleichzeitiger Formgebung zu Formkörpern, Imprägnierungen, Beschichtungen oder Verklebungen.

Ein besonderer Vorteil der erfindungsgemässen Zusammensetzungen liegt jedoch darin, dass sie schon bei besonders niedrigen Temperaturen im Bereich unter 140 °C, insbesondere zwischen 120 und 140 °C, mit für die Praxis ausreichender Geschwindigkeit ausgehärtet werden können. Die Gelierzeiten erfindungsgemässer Zusammensetzungen liegen nämlich im allgemeinen auch bei diesen Temperaturen unter 30 Minuten.

Beispiel 1:

Herstellung von

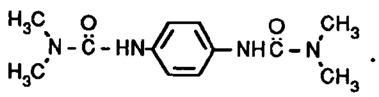

In einem 2-Liter-Rundkolben mit Rückflusskühler, Stickstoffein- und -auslass werden 112,09 g (0,7 Mol) 1,4-Phenylendiisocyanat (Ex Akzo Chem. Co.; heute z. B. bei Aldrich oder Fluka erhältlich) und 1,6 Liter Essigsäureethylester (Essigester) unter Rückfluss erhitzt. Nun werden 63,1 g (1,4 Mol) Dimethylamin eingeleitet. Man lässt auf Raumtemperatur abkühlen und filtriert die weisse Suspension durch eine G4-Nutsche. Nach Waschen mit Essigester wird das Produkt 14 Stunden lang bei 110 °C im Hochvakuum getrocknet. Man erhält 174 g (99,3 % der Theorie) des gewünschten Produkts.

Schmelzpunkt: >300 °C.

| | | | | |
|---|---|---|---|---|
| Elementaranalyse: | ber. | C 57,58% | H 7,25% | N 22,38% |
| | gef. | C 57,50% | H 7,24% | N 22,03% |

IR (KBr): ν(C=O) bei 1640 $cm^{-1}$; keine -NCO-Bande (ν ~ 2280 $cm^{-1}$) sichtbar.

$^1$H-NMR (DMSO-$d_6$): 2,90 ppm, s, 12H; 7,27 ppm, s, 4H; 8,06 ppm, s, 2H.

Beispiel 2:

Herstellung von

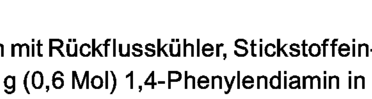

In einem 1,5-Liter-Sulfierkolben mit Rückflusskühler, Stickstoffein- und -auslass, Thermometer und Tropftrichter wird eine Lösung von 64,88 g (0,6 Mol) 1,4-Phenylendiamin in 600 Milliliter Pyridin vorgelegt und tropfenweise 141,95 g (1,32 Mol) Dimethylcarbamoylchlorid zugegeben. Es findet eine exotherme Reaktion statt. Man lässt 12 Stunden bei 100 °C reagieren, kühlt danach ab und giesst das Reaktionsgemisch auf eine Mischung aus Eis und 1N Salzsäure. Es wird durch eine G4-Nutsche filtriert, mit Wasser, danach mit Ethanol und Essigester gewaschen und 14 Stunden lang bei 50 °C im Hochvakuum getrocknet. Man erhält 116,8 g

(77,8 % der Theorie) des gewünschten Produkts in Form eines violetten Pulvers.
Schmelzpunkt: >300 °C.

| Elementaranalyse: | ber. | C 57,58% | H 7,25% | N 22,38% |
|---|---|---|---|---|
| | gef. | C 57,56% | H 7,23% | N 22,53% |

$^1$H-NMR (DMSO-$d_6$): 2,90 ppm, s, 12H; 7,28 ppm, s, 4H; 8,10 ppm, s, 2H.

Beispiel 3:

Herstellung von

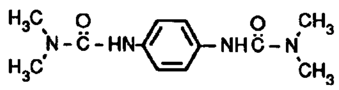

analog zur US-A-2,993,044.

In einem 6,3-Liter-Stahlautoklaven mit mechanischer Rührung (Ankerrührer) werden 136,08 g (1,26 Mol) 1,4-Phenylendiamin, 161,28 g (5,04 Mol) Schwefel und 315 Milliliter Methanol vorgelegt. Danach werden 340,2 g (7,56 Mol) Dimethylamin zugepresst, gefolgt von Kohlenmonoxid, bis ein Druck von 22 bar im Autoklaven herrscht. Die Temperatur steigt dabei von anfänglich 18 °C auf 36 °C an. Innerhalb von 75 Minuten fällt der Druck auf 10 bar. Man erhitzt auf 100 °C und belässt 2 Stunden bei dieser Temperatur (Druck 10 bar). Danach wird auf Raumtemperatur abgekühlt, der Autoklav mit Methanol ausgewaschen, die erhaltene schwarze Suspension eine Stunde lang auf 60 °C erhitzt und heiss filtriert. Der Rückstand wird mit heissem Methanol gewaschen, bis die Waschflüssigkeit farblos abläuft, und 14 Stunden lang bei 110 °C im Hochvakuum getrocknet. Man erhält 296 g (94 % der Theorie) des gewünschten Produkts.

| Elementaranalyse: | ber. | C 57,58% | H 7,25% | N 22,38% |
|---|---|---|---|---|
| | gef. | C 57,42% | H 7,28% | N 22,38% |

IR (KBr): ν(C=O) bei 1640 $cm^{-1}$
$^1$H-NMR (DMSO-$d_6$): 2,99 ppm, s, 12H; 7,44 ppm, s, 4H; 8,23 ppm, s, 2H.

Die Produkte der Beispiele 1,2 und 3 haben alle den gleichen Beschleunigungseffekt und das gleiche Latenzverhalten in den hierin beschriebenen Zusammensetzungen.

Beispiel 4:

Eine Mischung aus jeweils 20 g Epoxidharz auf Basis von Bisphenol A (Epoxidäquivalent 185 - 190 g(Äqu; Viskosität(bei 25 °C nach DIN 53015): 10000 - 12000 mPa·s; Araldit®GY-250); 2 g Dicyandiamid und 1 g Beschleuniger aus Beispiel 1 wird zur Homogenisierung zweimal über den Dreiwalzenstuhl gelassen. Diese Mischung hat die in Tabelle 1 angegebenen Eigenschaften (Gemisch A). Zum Vergleich sind in der Tabelle auch die Eigenschaften einer sonst identischen Mischung, die jedoch anstatt des erfindungsgemässen Härtungsbeschleunigers die gleiche Menge Chlortoluron als Beschleuniger enthält, (Gemisch B), sowie die Eigenschaften einer entsprechenden Mischung ohne jeglichen Beschleunigerzusatz (Gemisch C) aufgeführt.

Tabelle 1:

| Gemisch | Exothermie-Maximum[1] | ΔH [J/g] | Stabilität[2] bei 40°C | Zugscherkraft[3] (Al/Al) nach ISO 4587 | Gelierzeit in Minuten bei | | | |
|---|---|---|---|---|---|---|---|---|
| | | | | | 100°C | 120°C | 140°C | 160°C |
| A | 132°C | 400 | >100Tge | 19,4N/mm$^2$ | >60 | 23 | 6 | 3 |
| B | 149°C | 300 | < 14Tge | 17,9N/mm$^2$ | >60 | 11,5 | 5 | 1,25 |
| C | 202°C | 443 | >100Tge | 19,5N/mm$^2$ | >60 | >60 | >60 | 31 |

[1] bestimmt mit DSC bei 10°C/Minute Aufheizgeschwindigkeit
[2] Tge =Tage, bis eine Verdoppelung der Viskosität des Gemisches auftritt
[3] nach 60-minütiger Härtung bei 160°C

Bei einem Vergleich der für die Gemische A und B angegebenen Daten erkennt man deutlich die stark gestiegene Stabilität der erfindungsgemässen Zusammensetzung (Gemisch A) gegenüber einer Zusammensetzung mit Chlortoluron (Gemisch B), obwohl beide Zusammensetzungen bei erhöhter Temperatur annähernd gleich reaktiv sind und demzufolge bei diesen Temperaturen praktisch gleich schnell gelieren und auch härten. Ein Vergleich der Werte für die Gemische A und C lässt weiterhin erkennen, dass die Stabilität der erfindungsgemäss beschleunigten heisshärtenden Zusammensetzung A sogar vergleichbar ist mit einer unbeschleunigten, sonst aber identischen Zusammensetzung (Gemisch C).

Beispiel 5:

Die folgende Klebstoff-Mischung (Gemisch D) wird zweimal zur Homogenisierung über den Dreiwalzenstuhl gelassen:

9,52 g Bisphenol-A-diglycidylether
2,36 g 1,4-Butandiol-diglycidylether
0,34 g roher ungereinigter Bisphenol-A-diglycidylether (5,2 - 5,4 Äquivalente Epoxid/Kilogramm)
6,00 g Addukt aus Bisphenol-A-diglycidylether/carboxylterminiertem Butadien-Acrylonitril-Copolymer/Cashew nut shell oil (3,0 - 3,5 Äquivalente Epoxid/Kilogramm)
0,70 g Dicyandiamid
0,60 g Siliziumdioxid
0,48 g eines Beschleunigers gemäss den Beispielen 1 - 3

Zum Vergleich wird eine identische Mischung, die jedoch die identische Menge Chlortoluron als Beschleuniger enthält, in der gleichen Weise homogenisiert (Gemisch E).

In Tabelle 2 sind wichtige Eigenschaften beider Mischungen gegenübergestellt.

Tabelle 2:

| Gemisch | Exothermie-Maximum[1] | ΔH [J/g] | Stabilität[2] bei 40°C | Zugscherkraft[3] (Al/Al) nach ISO 4587 | Gelierzeit in Minuten bei | | | |
|---|---|---|---|---|---|---|---|---|
| | | | | | 100°C | 120°C | 140°C | 160°C |
| D | 157°C | 252 | >100Tge | 30,3N/mm$^2$ | >60 | 29 | 8,3 | 2,3 |
| E | 152°C | 237 | < 30Tge | 35,6N/mm$^2$ | >60 | 19 | 8,2 | 3,5 |

[1] bestimmt mit DSC bei 10°C/Minute Aufheizgeschwindigkeit
[2] Tge =Tage, bis eine Verdoppelung der Viskosität auftritt
[3] nach 60-minütiger Härtung bei 160°C

Wieder tritt das gestiegene Stabilitäts-/Reaktivitätsverhältnis der erfindungsgemässen Zusammensetzung D deutlich zu Tage.

## Patentansprüche

1. Verwendung einer Verbindung der Formel 1

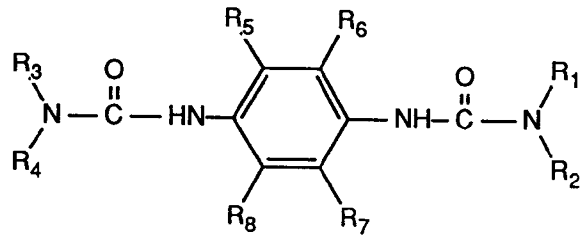

(1),

worin
$R_1$ und $R_3$ unabhängig voneinander jeweils eine Alkyl-, Cycloalkyl- oderArylgruppe,
$R_2$ und $R_4$ unabhängig voneinander jeweils eine Alkyl- oder Arylgruppe oder
$R_1$ mit $R_2$ und/oder $R_3$ mit $R_4$ jeweils eine 1,4-Tetramethylen- oder eine 1,5-Pentamethylengruppe und schliesslich
$R_5$, $R_6$, $R_7$ und $R_8$ unabhängig voneinander jeweils ein Wasserstoff- oder Halogenatom oder eine $C_1$-$C_4$-Alkyl- oder $C_1$-$C_4$-Alkoxygruppe bedeuten,
als Härtungsbeschleuniger für eine heisshärtende Zusammensetzung enthaltend mindestens ein Epoxidharz und Dicyandiamid.

2. Heisshärtende Zusammensetzung enthaltend mindestens ein Epoxidharz, Dicyandiamid und einen Härtungsbeschleuniger, dadurch gekennzeichnet, dass als Härtungsbeschleuniger eine Verbindung der Formel 1 eingesetzt wird:

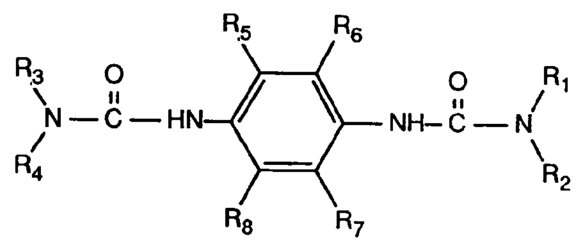

(1),

worin
$R_1$ und $R_3$ unabhängig voneinander jeweils eine Alkyl-, Cycloalkyl- oder Arylgruppe,
$R_2$ und $R_4$ unabhängig voneinander jeweils eine Alkyl- oder Arylgruppe oder
$R_1$ mit $R_2$ und/oder R3 mit $R_4$ jeweils eine 1,4-Tetramethylen- oder eine 1,5-Pentamethylengruppe und schliesslich
$R_5$, $R_6$, $R_7$ und $R_8$ unabhängig voneinander jeweils ein Wasserstoff- oder Halogenatom oder eine $C_1$-$C_4$-Alkyl oder $C_1$-$C_4$-Alkoxygruppe bedeuten.

3. Zusammensetzung nach Anspruch 2, dadurch gekennzeichnet, dass $R_1$, $R_2$, $R_3$ und $R_4$ in Formel 1 unabhängig voneinander eine Alkylgruppe mit 1 bis 3 Kohlenstoffatomen oder Phenyl bedeuten.

4. Zusammensetzung nach einem der Ansprüche 2 oder 3, dadurch gekennzeichnet, dass die Reste $R_5$, $R_6$, $R_7$ und $R_8$ in Formel 1 alle Wasserstoffatome darstellen.

5. Zusammensetzung nach Anspruch 4, dadurch gekennzeichnet, dass $R_1$, $R_2$, $R_3$ und $R_4$ in Formel 1 alle Methyl bedeuten.

6. Zusammensetzung nach einem der Ansprüche 2 bis 5, dadurch gekennzeichnet, dass sie zusätzlich ein Polyisocyanat enthält.

7. Zusammensetzung nach einem der Ansprüche 2 bis 6, dadurch gekennzeichnet, dass sie die Verbindung der Formel 1 in einer Menge von 0,1 bis 10 Gewichtsprozent enthält, bezogen auf die Gesamtmenge aus

Epoxidharz sowie Dicyandiamid und gegebenenfalls vorhandenem Polyisocyanat.

8. Verfahren zur Herstellung von Verbindungen der Formel 1 gemäss Anspruch 1, wobei man eine Diisocyanatverbindung der Formel

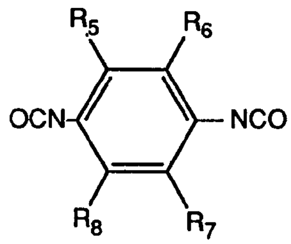 ,

worin die Reste $R_5$, $R_6$, $R_7$ und $R_8$ die gleiche Bedeutung wie in Anspruch 1 haben, mit jeweils zumindest etwa stöchiometrischen Mengen der Amine $HNR_1R_2$ und $HNR_3R_4$, wobei die Reste $R_1$ bis $R_4$ ebenfalls die in Anspruch 1 angegebene Bedeutung haben, in einem geeigneten Lösungsmittel unter Erwärmen umsetzt und das erhaltene Produkt der Formel 1 isoliert.

9. Verfahren zur Herstellung von Verbindungen der Formel 1 gemäss Anspruch 1, wobei man eine Diaminoverbindung der Formel

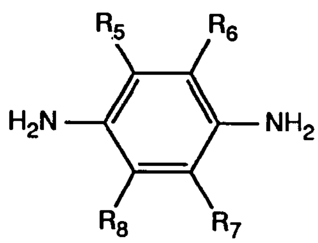 ,

worin die Reste $R_5$, $R_6$, $R_7$ und $R_8$ die gleiche Bedeutung wie in Anspruch 1 haben, mit zumindest etwa stöchiometrischen Mengen der Carbamoylchloride der Formeln

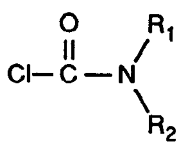 und 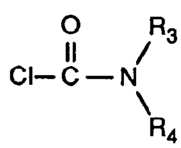 ,

wobei die Reste $R_1$ bis $R_4$ ebenfalls die in Anspruch 1 angegebene Bedeutung haben, in einem geeigneten Lösungsmittel umsetzt und das erhaltene Produkt von Formel 1 isoliert.

Europäisches Patentamt

# EUROPÄISCHER RECHERCHENBERICHT

Nummer der Anmeldung
EP 93 81 0861

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| P,X | JOURNAL OF APPLIED POLYMER SCIENCE, Bd.47, Nr.8, 20. Februar 1993, NEW YORK US Seiten 1401 - 1409 P. J. PEARCE ET AL. 'Aging and performance of structural film adhesives.' * das ganze Dokument * --- | 1-7 | C07C275/40 C08G59/68 C08G59/40 |
| X,Y | EP-A-0 429 395 (CIBA-GEIGY AG) * Seite 4, Zeile 19, 24; Anspruch 1 * --- | 1-7 | |
| Y | EP-A-0 344 468 (SCHERING AG) * Ansprüche 1-9 * --- | 1-7 | |
| X | EP-A-0 108 712 (CIBA-GEIGY AG) * Seite 4, Zeile 7 - Seite 5, Zeile 4 * --- | 8,9 | |
| X | EP-A-0 138 769 (CIBA-GEIGY AG) * Seite 4 - Seite 15 * --- | 8,9 | |
| A | US-A-3 386 956 (ALEKSANDRA CHROBOK NAWAKOWSKI ET AL.) * das ganze Dokument * ----- | 1-9 | RECHERCHIERTE SACHGEBIETE (Int.Cl.5) C07C C08G |

Der vorliegende Recherchenbericht wurde für alle Patentansprüche erstellt

| Recherchenort | Abschlußdatum der Recherche | Prüfer |
|---|---|---|
| BERLIN | 11. März 1994 | Rufet, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)